# EUROPEAN PATENT APPLICATION

(11) **EP 2 233 145 A1**
(43) Date of publication of application: **29.09.2010**
(21) Application number: 09290202.2
(22) Date of filing: 19.03.2009
(51) Int. Cl.: A61K 31/727, A61K 31/765, A61P 9/00, A61P 7/02, A61P 13/12, A61P 41/00

(54) **A dose of AVE5026 for the treatment of venous thromboembolism in patients with severe renal impairment**

(71) Applicant: Sanofi-Aventis, 75013 Paris (FR)
(72) Inventor: Clot, Pierre-François, 75013 Paris (FR); Dubruc, Catherine, 75013 Paris (FR)
(74) Representative: Romanowski, Caroline

(57) **Abstract**

The invention relates to a dose of 10 mg of AVE5026 for the treatment of venous thromboembolism in patients with severe renal impairment.

## Description

The invention relates to a specific dose of AVE5026 for the treatment of venous thromboembolism in patients with severe renal impairment.

AVE5026 (sanofi-aventis laboratory code) belongs to a new generation of hemisynthetic heparins. It is a new ultra-low molecular weight heparin, with an average molecular weight of 2000-3000 Daltons and a novel antithrombotic profile resulting from high anti-Factor Xa activity and residual anti-Factor IIa activity. It is obtained by selective and controlled depolymerization of heparin, as described in the patent application WO 2004/033503.

AVE5026 is in clinical development for venous thromboembolism (VTE) prevention (see The Pink Sheet, October 1st, 2007, Vol. 69, No. 040, page 19). In the phase II TREK study, compared to enoxaparin (low molecular weight heparin commercialized under the tradename Lovenox^{®} or Clexane^{®}), 20 mg and 40 mg doses of AVE5026 showed a superior efficacy for confirmed adjudicated VTE, with a good safety profile. The 20 mg dose of AVE5026 has been selected for future investigation in clinical studies of VTE prevention.

In the field of therapy with low molecular weight heparin products, patients with severe renal impairment are known to have a higher bleeding risk. Indeed, they have an increased exposure to the drug due to their impaired renal function.

The Applicant has now found that a specific dose of AVE5026, namely 10 mg, is effective and safe in patients with severe renal impairment.

The subject-matter of the invention is therefore a dose of 10 mg of AVE5026 for use in therapy in patients with severe renal impairment.

According to the present invention, the terms below have the following meanings:
- "therapy" means either a curative treatment or a prophylactic treatment with the drug AVE5026;
- "treatment" means a venous thromboprophylactic treatment to a patient for whom thromboprophylaxis is indicated;
- "patients with severe renal impairment" designates patients for whom their creatinine clearance value is lower than 30 mL/min.

Advantageously, the therapeutic regimen of the present invention does not require subsequent monitoring and dose adjustment.

In an embodiment, the invention relates to the above dose of AVE5026 for a once a day administration.

In another embodiment, the invention relates to the above dose of AVE5026 for administration by the subcutaneous route.

In another embodiment, the invention relates to a dose of 10 mg of AVE5026 for patients with severe renal impairment, for the prophylaxis of venous thromboembolism. More specifically, the invention relates to the above dose of AVE5026 for patients with severe renal impairment for prophylaxis of deep venous thrombosis (DVT) which may lead to pulmonary embolism (PE).

In another embodiment, the invention relates to a dose of 10 mg of AVE5026 in patients with severe renal impairment, for the prophylaxis of venous thromboembolism:
- in patients undergoing knee replacement surgery, hip replacement surgery or hip fracture surgery, or
- in patients undergoing abdominal surgery who are at risk for thromboembolic complications.

The invention also relates to the use AVE5026 at a 10 mg dose for the preparation of a medicament for use in therapy, more specifically for the prophylaxis of venous thromboembolism, in patients with severe renal impairment. All the embodiments described above also apply to said use of AVE5026.

The invention also relates to a pharmaceutical composition comprising, as active ingredient, AVE5026 at a 10 mg dose, and at least one pharmaceutically acceptable excipient. Said excipients are chosen according to the pharmaceutical form and the administration route desired, among usual excipients known to one of skill in the art.

The pharmaceutical composition according to the invention is preferably an injectable solution adapted to the subcutaneous route.

Having now described the present invention in detail, the same will be more clearly understood by reference to the following examples of the invention, which are included herewith for purposes of illustration only and are not intended to be limiting of the invention.

### 1) Preparation of AVE5026

AVE5026 is prepared by depolymerization of a quaternary ammonium salt of the benzyl ester of heparin in an organic medium, by means of a phosphazene base (BEMP: 2-tert-butylimino-2-diethylamino-1,3-dimethylperhydro-1,2,3-diazaphosphorine), conversion of the quaternary ammonium salt of the benzyl ester of the depolymerized heparin to a sodium salt, saponification of the residual esters and finally purification.

The depolymerization step is advantageously performed in an aprotic solvent, such as dichloromethane, containing a percentage of water of less than 0.6%. Preferably, this percentage of water should be chosen less than 0.3% and most particularly less than 0.2%.

Advantageously, the phosphazene base/ester mol ratio is between 0.2 and 5, preferably between 0.6 and 2 and most particularly between 0.8 and 1.2. The use of the equimolar ratio is preferred.

The quaternary ammonium salt of the benzyl ester of heparin is advantageously the benzethonium salt.

The method for preparing the AVE5026 therefore comprises the following steps:
a) transalification of sodium heparin by the action of benzethonium chloride,
b) esterification of benzethonium heparinate by the action of benzyl chloride,
c) transalification of the heparin benzyl ester obtained to a quaternary ammonium salt, by benzethonium salts,
d) depolymerization of the benzethonium salt of the benzyl ester of heparin by the method as defined above,
e) conversion of the benzethonium salt to a sodium salt,
f) saponification by the action of a base such as sodium hydroxide,
g) purification, in particular by the action of an oxidizing agent such as hydrogen peroxide.

Step e) is generally carried out by treating the reaction medium with an alcoholic solution of sodium acetate and preferably with a 10% solution of sodium acetate in methanol (weight/volume), at a temperature of between 15 and 25°C. The equivalent by weight of acetate added is preferably 3 times greater than the mass of benzethonium salt of the benzyl ester of heparin used in the depolymerization reaction.

The saponification (step f) is generally carried out by means of an alkali metal hydroxide such as sodium hydroxide, potassium hydroxide or lithium hydroxide, in an aqueous medium, at a temperature of between 0 and 20°C and preferably between 0 and 10°C. 1 to 5 molar equivalents of alkali metal hydroxide will be generally used. Preferably, the saponification will be carried out in the presence of 1 to 2 molar equivalents of alkali metal hydroxide.

The purification (step g) is carried out by means of hydrogen peroxide, in an aqueous medium, at a temperature of 10 to 50°C. Preferably, this operation is carried out between 20 and 40°C.

The quaternary ammonium salt (benzethonium salt) of the benzyl ester of heparin is prepared according to the following reaction scheme:
a) conversion of the heparin to the form of a sodium salt by means of benzethonium chloride in order to obtain benzethonium heparinate (transalification),
b) esterification of the benzethonium salt obtained above by means of benzyl chloride and treatment with an alcoholic solution of sodium acetate in order to obtain the sodium salt of the benzyl ester of heparin, and
c) transalification of the sodium salt of the benzyl ester of heparin to a quaternary ammonium salt (benzethonium salt).

The reaction of step a) is carried out by the action of benzethonium chloride in excess, on sodium heparin, at a temperature in the region of 15 to 25°C. Advantageously, the salt/sodium heparin molar ratio is between 3 and 4.

The starting heparin used is preferably a pig heparin. The latter may be purified beforehand in order to reduce its dermatan sulfate level according to the method described in patent FR2663639.

The esterification of step b) is preferably carried out in a chlorinated organic solvent (for example chloroform or methylene chloride), at a temperature of between 25 and 45°C and preferably between 30 and 40°C. The ester in the form of a benzethonium salt is then recovered in the form of a sodium salt by precipitation by means of sodium acetate at 10% by weight in an alcohol such as methanol. 1 to 1.2 volumes of alcohol are generally used per volume of reaction medium. The quantity of benzyl chloride and the reaction time are adjusted in order to obtain a degree of esterification of between 50 and 100% and preferably between 70 and 90%. Preferably, 0.5 to 1.5 parts by weight of benzyl chloride are used for 1 part by weight of benzethonium salt of heparin. Likewise, preferably the reaction time will be between 10 and 35 hours.

The transalification step c) is carried out by means of benzethonium chloride in an aqueous medium, at a temperature of between 10 and 25°C. Advantageously, the quaternary ammonium chloride/sodium salt of the benzyl ester of heparin mol ratio is between 2 and 3.

This process, described in WO 2004/033503, enables to obtain a low molecular weight heparin with an average molecular weight of 2000-3000 Daltons, an anti-Factor Xa activity of between 150-200 IU/mg (most particularly, between 150-180 IU/mg), an anti-Factor IIa activity of less than 5 IU/mg, and most particularly of 0.5 to 3.5 IU/mg, and an anti-Factor Xa/anti-Factor IIa activity ratio of greater than 30. Given its very low molecular weight, such a product is also called an "ultra-low" molecular weight heparin.

The anti-Xa activity is measured by the amidolytic method on a chromogenic substrate described by Teien et al., Thromb. Res., 10, 399-410 (1977), with, as standard, the first international standard for low molecular weight heparins. The anti-IIa activity is measured by the technique described by Anderson L.O. et al., Thromb. Res., 15, 531-541 (1979), with, as standard, the first international standard for low molecular weight heparins.

### 2) Pharmaceutical composition comprising AVE5026

AVE5026 is formulated as an injectable solution, adapted to the subcutaneous route.

An example of such a formulation is as follows: 0.2 mL of a sterile, isotonic solution of a 50 mg/mL AVE5026 solution in water for injection with sodium chloride 0.9%.

The pharmaceutical composition of AVE5026 may be presented in a ready-to-use pre-filled syringe.

### 3) Dosage in SRI patients

### 3.1: POP6240 study

This study was performed for assessing the pharmacokinetics (PK) and safety on humans with renal impairment (RI), by comparison with normal renal function subjects, after repeated subcutaneous administration of AVE5026 once daily, for 7 days. The daily doses of AVE5026 were either 40 mg (subjects with normal renal function or with mildly or moderately impaired renal function) or 20 mg (subjects with severe renal impairment).

Renal function groups were defined according to creatinine clearance (CLcr) values calculated using the well-known Cockroft-Gault formula, and were classified according to the following characteristics :
- Renal function Group I: 8 subjects with normal renal function (CLcr > 80 mL/min);
- Renal function Group II: 8 subjects with mild RI (50 ≤ CLcr ≤ 80 mL/min);
- Renal function Group III: 8 subjects with moderate RI (30 ≤ CLcr < 50 mL/min);
- Renal function Group IV: 8 subjects with severe RI (CLcr < 30 mL/min, but not requiring hemodialysis).

The following PK parameters were determined based on anti-human blood coagulation factor Xa (anti-Xa) activity:
- Maximum plasma concentration observed (Cₘₐₓ), area under the plasma concentration versus time curve from time 0 to time 24 hours (AUC₀₋₂₄), and time at maximal plasma concentrated observed (tₘₐₓ) on Days 1 and 7;
- Apparent plasma clearance (CUF), apparent distribution volume at steady state (Vss/F), and apparent terminal half-life (t_{1/2z}) on Day 7;
- Accumulation ratios (Rac): Cₘₐₓ (Day 7)/Cₘₐₓ (Day 1), AUC₀₋₂₄ (Day 7)/ AUC₀₋₂₄ (Day 1).

In subjects with mild RI, exposure parameters were similar to those observed in normal subjects on Day 1 and Day 7, with similar Rac.

In subjects with moderate RI, AUC₀₋₂₄ was higher on Day 1 and on Day 7 than in normal subjects. In these subjects, the terminal half-life was longer than in normal subjects, with a higher Rac for AUC₀₋₂₄.

In subjects with severe RI, receiving only 20 mg (half of that in normal subjects and other groups) of AVE5026, the AUC₀₋₂₄ on Day 7 was lower than that in normal subjects, the terminal half-life was longer and the Rac for AUC₀₋₂₄ was higher. Renal excretion represented the lowest fraction of the AVE5026 dose in subjects with severe RI, compared to subjects with normal, mild and moderate RI.

### 3.2: Dosage adjustment in SRI patients

The dosage adjustment was based on the results of the above phase I study (POP6240) and on PK predictions. Pharmacokinetics analyses performed on a pool of Phase I studies, including the POP6240 study, have shown that creatinine clearance (CLcr) was the main covariate affecting AVE5026 clearance. The population pharmacokinetic model built on the pool of Phase I studies was used to predict typical patients with demographic values set to the median values of the TREK phase II patient population. These simulations enabled to select the 10 mg dose for SRI patients, allowing an exposure to the drug close to that of patients with normal renal function, while favouring the safety side.

For patients with mild and moderate renal impairment, the population pharmacokinetic model built on the pool of phase I studies showed that the same dose than that administered to normal renal function patients could be used safely.

## Claims

1. A dose of 10 mg of AVE5026 for use in therapy in patients with severe renal impairment.

2. The dose according to claim 1, for a once a day administration.

3. The dose according to claim 1 or claim 2, for administration by the subcutaneous route.

4. The dose according to any of claims 1 to 3, for the prophylaxis of venous thromboembolism.

5. The dose according to any of claims 1 to 4, for the prophylaxis of deep venous thrombosis.

6. The dose according to any of claims 1 to 5, for the prophylaxis of deep venous thrombosis which may lead to pulmonary embolism.

7. The dose according to any of claims 1 to 6, for the prophylaxis of venous thromboembolism in patients undergoing knee replacement surgery, hip replacement surgery or hip fracture surgery, or in patients undergoing abdominal surgery who are at risk for thromboembolic complications.

8. Use of AVE5026 at a 10 mg dose for the preparation of a medicament for use in therapy in patients with severe renal impairment.

9. A pharmaceutical composition comprising, as active ingredient, AVE5026 at a 10 mg dose, and at least one pharmaceutically acceptable excipient.
